# EUROPEAN PATENT APPLICATION

(11) **EP 2 210 627 A2**
(43) Date of publication of application: **28.07.2010**
(21) Application number: 10158668.3
(22) Date of filing: 18.03.2003
(51) Int. Cl.: A61L 31/18, A61L 31/14

(54) **MRI and X-RAY Compatible Stent Material**

(30) Priority: 22.03.2002 US 63125
(62) Divisional of application: 03745540.9
(71) Applicant: Boston Scientific Limited, Christ Church (BB)
(72) Inventor: Jansen, Lex P., Pleasanton, CA 94566 (US); Griffin, Stephen, Sunnyvale, CA 94086 (US)
(74) Representative: Peterreins, Frank

(57) **Abstract**

A medical implant for implantation in a bodily vessel may comprise a tungsten-rhenium (Re) alloy. Another medical implant for implantation in a bodily vessel may comprise molybdenum TZM alloy. Another medical implant may be provided in the form of a stent manufactured from a sheet of metal or from a metal tube, the metal having a modulus of elasticity of 300 GPa or greater.

## Description

### Background of Invention

Stents may be placed within the body to maintain and/or treat a body lumen that has been impaired or occluded. Stents are typically made from strands of material formed into a tube, of rolled sheets of material with openings or of tubes with material removed therefrom to form a stent pattern. Stents are usually delivered into the body lumen using a catheter. The catheter carries the stent to the desired site and the stent is released from the catheter and expands to engage the inner surface of the lumen. Placement of the stent may be monitored by fluoroscopy where the stent includes radiopaque materials.

Stents may be balloon expandable, self-expanding or a combination of self-expanding and balloon expandable. Self-expanding stents are made with a material having a restoring force. Suitable materials for self-expanding stents include spring steel and shape memory materials such as Nitinol. Balloon expandable stents are often made with elastic, plastically deformable materials such as stainless steel. Other suitable materials for stents include cobalt chrome alloys such as elgiloy, tantalum and other plastically deformable metals, platinum/tungsten alloys and titanium alloys.

Because stents are delivered through often tortuous vessels, they must be flexible. In order to achieve flexible and expandable stents, the stent material desirably should have a high modulus of elasticity. At the same time, the materials should be sufficiently radiopaque to be visible under fluoroscopy. Moreover, with the widespread use of Magnetic Resonance Imaging (MRI), it is desirable for the stent material to be MRI compatible so as not to distort any magnetic resonance images that may be taken of the body in the region of the stent.

Similar material considerations also apply to other types of medical implants, including, for example, vena cava filters as well as other implant devices which require high strength as well as resistance to fracture so that the device may be deployed without fracturing.

There remains a need for stent materials and other implant materials which have high modulii of elasticity, are radiopaque and which are MRI compatible.

All US patents and applications and all other published documents mentioned anywhere in this application are incorporated herein by reference in their entirety.

Without limiting the scope of the invention a brief summary of some of the claimed embodiments of the invention is set forth below. Additional details of the summarized embodiments of the invention and/or additional embodiments of the invention may be found in the Detailed Description of the Invention below.

A brief abstract of the technical disclosure in the specification is provided as well for the purposes of complying with 37 C. F. R. 1.72.

### Summary of Invention

In one embodiment, the invention is directed to a medical implant for implantation in a bodily vessel where the implant comprises a tungsten-rhenium alloy.
The implant has a flow passage therethrough and, desirably, is in the form of a stent or vena cava filter. The implant may optionally be manufactured from a tungsten-rhenium alloy consisting essentially of tungsten and rhenium. Typically, tungsten will be present in an amount ranging from about 75 weight percent to about 99 weight percent and rhenium will be present in an amount ranging from about 25 weight percent to about 1 weight percent. The implant may be manufactured from a sheet of material or from a tube or from other suitable forms of the alloy.

In another embodiment, the invention is directed to a medical implant for implantation in a bodily vessel where the implant comprises molybdenum TZM alloy. The implant has a flow passage therethrough and, desirably, is in the form of a stent. Or vena cava filter. Optionally, the medical implant may be constructed of a molybdenum TZM alloy having a modulus of elasticity of at least 300 GPa. The implant may be manufactured from a sheet of material or from a tube or from other suitable forms of the alloy.

In another embodiment, the invention is directed to a stent manufactured from a sheet of metal or from a metal tube or other form of the alloy where the metal has a modulus of elasticity of 300 GPa or greater. The stent may be manufactured from a sheet of material or from a tube or from other suitable forms of the alloy. The stent may comprise a tungsten-rhenium alloy, a molybdenum TZM alloy or may consist essentially of molybdenum. In the case of a tungsten-rhenium alloy, the tungsten is desirably present in an amount ranging from about 75 weight percent to about 99 weight percent and the rhenium is desirably present in an amount ranging from about 25 weight percent to about 1 weight percent. Optionally, the stent may have modulus of elasticity of 400 GPa or greater.

In another embodiment, the invention is directed to a medical implant comprising molybdenum TZM alloy. Typically, the medical implant will be in the form of a stent.

Additional details and/or embodiments of the invention are discussed below.

### Brief Description of Drawings

FIG. 1 is a perspective view of an implant in the form of a stent.
FIG. 2 is a perspective view of the stent of Fig. 1 in an expanded state.
FIG. 3 is a side view of another implant in the form of a stent.
FIG. 4 is a flat pattern of the stent of Fig. 3.

### Detailed Description

While this invention may be embodied in many different forms, there are described in detail herein specific desirable embodiments of the invention. This description is an exemplification of the principles of the invention and is not intended to limit the invention to the particular embodiments illustrated.

For the purposes of this disclosure, unless otherwise indicated, identical reference numerals used in different figures refer to the same component.

The invention disclosed herein is directed to medical implants. Typically, the medical implant will be in the form of a device having a flowpath therethrough.
Desirably, the implant will be in the form of a stent although it may also be provided in other forms as well, for example, as a vena cava filter.

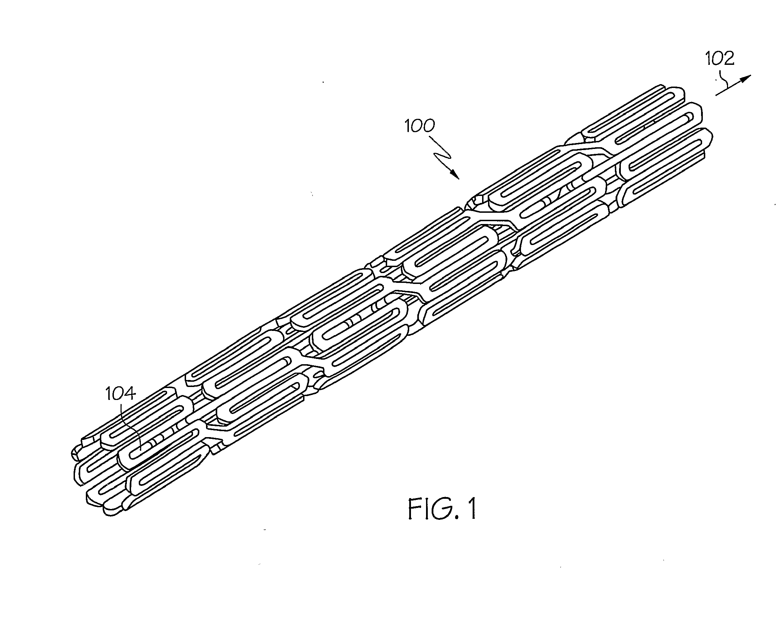

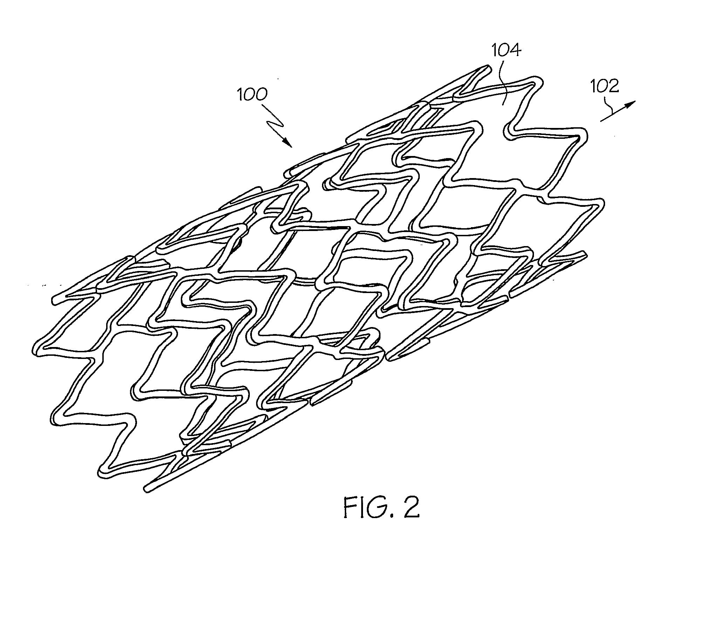

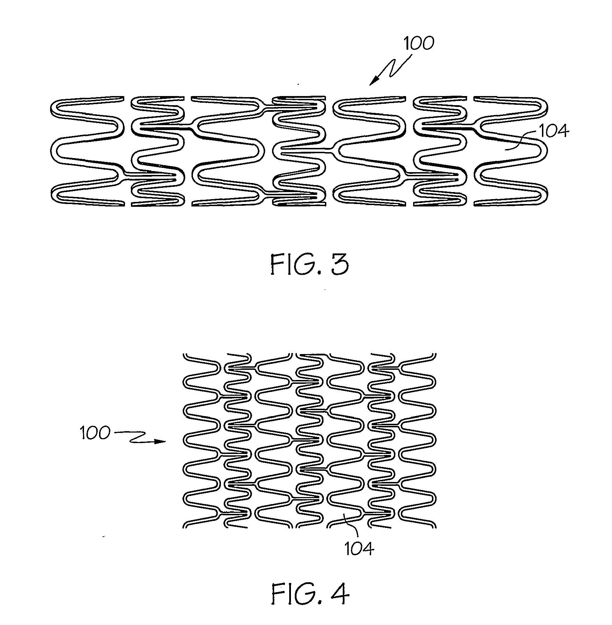

An example of a stent is shown generally in an unexpanded state at 100 in Fig. 1. Stent 100 has a flowpath therethrough in the direction of the longitudinal axis 102 of the stent. The stent has a sidewall with a plurality of openings 104 therethrough. The stent of Fig. 1 is shown in an expanded state in Fig. 2. Another stent design is shown generally at 100 in Figs. 3 and 4. The stent designs of Figs. 1-4 are shown for exemplary purposes only. The inventive stents disclosed herein may be manufactured in any other known stent geometry.

The stent may be manufactured using any suitable stent manufacturing technique. The stent may be manufactured from a sheet of metal, from a tube or may be made from individual cylinders which are joined together. In the case of a stent manufactured from a sheet of metal, the sheet may be rolled and the edges of the metal may optionally be secured to another or may be left unsecured to one another. The openings may be provided in the stent by chemically etching the sheet or tube, by laser cutting the tube or through any other known technique for providing such openings.

The invention may also be provided in the form of a vena cava filter. More information concerning vena cava filters may be found in US 5,059, 205 and in US 5,951,585.

In one aspect, the invention is directed to medical implants which use alloys that have not previously been used for implants. Desirably, the alloys are MRI compatible. Suitable alloys include molybdenum TZM, and tungsten-rhenium alloys. Pure molybdenum may be used in some embodiments of the invention.

In one embodiment, the invention is directed to medical implants such as those discussed above comprising molybdenum TZM alloy. Molybdenum TZM is a molybdenum-based alloy that contains small additions of finely dispersed particles, for example, between about 0.25% and 1.0% titanium by weight, and desirably 0.5% titanium, between about 0.04% and 2.0% zirconium by weight, and desirably 0.08% zirconium by weight, and between about 0.01 % to about 0.04% carbon by weight. Molybdenum TZM has been used as, for example, a vacuum furnace hot-zone material, a hot die and mold material, x-ray tube target and cathode cup material, or material for parts in numerous aerospace products.

Where the implant is in the form of a stent, a tube, at least a portion of which is of TZM alloy may be provided and a stent pattern provided therein by removing material from the tube. As discussed above, the material may be removed from the tube by etching, laser cutting or any other suitable materials removal process. Follow materials removal, the tube will have either of the patterns shown in Figs. 1-4 or any other desired pattern. The tube may then be polished using any suitable polishing process and/or plated or coated with a desired material, resulting in stent for implantation in the body.

The inventive TZM alloy stents may also be manufactured by assembling individual segments comprising TZM alloy. For example, one or more individual tubular segments made of TZM alloy may be joined together via welding, the use of adhesives or any other suitable method to produce a stent. The individual tubular segments may optionally be constructed to support vessels on their own or may be incapable, on their own, of supporting a vessel. Segments of other materials may also be included as part of the stent.

The stent may also be made from a sheet of TZM alloy having a materials removed therefrom to form a stent pattern or of a sheet, a portion of which is made of TZM alloy. The sheet may be rolled and two opposing edges secured to another to form a tube or the sheet may be left in a rolled configuration without securing two opposing edges to one another. The sheet may then be polished a needed and/or coated in part or in its entirety.

The invention is also directed, in other embodiments, to medical implants such as those discussed above comprising a tungsten-rhenium alloy. The implant may optionally be manufactured from a tungsten-rhenium alloy consisting essentially of tungsten and rhenium or may comprise a tungsten-rhenium alloy as well as other materials. Typically, in the inventive implants, tungsten will be present in an amount ranging from about 75 weight percent to about 99 weight percent and rhenium will be present in an amount ranging from about 25 weight percent to about 1 weight percent.
Desirably, if trace impurities are present, each impurity will be present in an amount not exceeding 1 percent by weight.

In one desirable embodiment, the tungsten-rhenium alloy consists essentially of tungsten in approximately 75 weight percent and rhenium in approximately 25 weight percent. In another desirable embodiment, the tungsten- rhenium alloy consists essentially of tungsten in approximately 95 weight percent and rhenium in approximately 5 weight percent. In another desirable embodiment, the tungsten-rhenium alloy consists essentially of tungsten in approximately 97 weight percent and rhenium in approximately 3 weight percent.

The entirety of the implant in general and stent in particular may be made of the tungsten-rhenium alloy or only a portion may be made of the alloy with the remainder of the implant made from one or more different materials.

Desirably, stents made from a tungsten-rhenium alloy, or those portions of a stent made from tungsten-rhenium alloy will have a modulus of elasticity of at least 400 GPa.

In another embodiment, the invention is directed to a medical implant such as those discussed above for implantation in a bodily vessel where the implant comprises molybdenum TZM alloy. The implant, desirably in the form of a stent, may be made using any of the other techniques disclosed herein for manufacturing a stent. The stent in its entirety may be made of the molybdenum TZM alloy or only selected portions of the stent may be made of the molybdenum TZM alloy.

Optionally, the medical implant may be constructed in part or in whole of a TZM alloy having a modulus of elasticity of at least 300 GPa.

In another embodiment, the invention is directed to an implant for a bodily vessel manufactured from a sheet of metal or from a metal tube where the metal has a modulus of elasticity of 300 GPa or greater. The implant, for example a stent or vena cava filter, may comprise a tungsten-rhenium alloy, a molybdenum TZM alloy or may consist essentially of molybdenum.

The entirety of the implant may be made with a tungsten-rhenium alloy, a molybdenum TZM alloy or molybdenum or only a portion of the implant may be made with these materials, with other portions of the implant made from other materials. The invention also contemplates implants in general, and stents in particular manufactured from a combination of tungsten-rhenium alloy, a molybdenum TZM alloy or molybdenum. As such, portions of the implant may be made of one of the materials and other portions may be made of other of the material.

The various portions may be joined together via welding, adhesively or via any other suitable joining technique. In one embodiment, one or more portions of the stent may be made of a molybdenum TZM alloy or tungsten-rhenium alloy with one modulus of elasticity and one or more other portions of the stent may be made of a molybdenum TZM alloy or tungsten- rhenium alloy having a different modulus of elasticity. As an example, one or both ends of the stent may be of a higher or lower modulus of elasticity than the middle portion of the stent. The stent may also be constructed and arranged to have an increasing modulus of elasticity along the length of the stent.

In the case of an implant comprising a tungsten-rhenium alloy, the tungsten is desirably present in an amount ranging from about 75 weight percent to about 99 weight percent and the rhenium is desirably present in an amount ranging from about 25 weight percent to about 1 weight percent.

Optionally, the stent may have a modulus of elasticity of 400 GPa or greater. Stents formed of tungsten-rhenium alloy typically have such an elasticity.

The invention is directed to any of the above stents whether in an unexpanded state or in an expanded state.

The invention is further directed to bifurcated stents where one or more portions of the bifurcated stent are made of any of the above alloys or molybdenum.
For example, each of the legs of a bifurcated stent or a single leg of the bifurcated stent or the entirety of the bifurcated stent may comprise any of the above disclosed tungsten-rhenium alloys or molybdenum TZM alloys or may be made entirely of molybdenum. Additional details concerning bifurcated stents may be found in US 5,916, 263 as well as in US Application No. 09/659571.

Any of the inventive stents disclosed above may be provided with a uniform diameter or may taper in portions or along the entire length of the stent. Also, thickness of the various portions of the inventive stents may increase or decrease along a given portion of the stent. For example, one or both ends of the stent may have a thicker wall or a thinner wall than the middle of the stent. The stent may also be provided in an embodiment in which the wall thickness increases along the length of the stent.

The inventive stents disclosed herein may find use in coronary arteries, renal arteries, peripheral arteries including illiac arteries, arteries of the neck and cerebral arteries. The stents of the present invention, however, are not limited to use in the vascular system and may also be advantageously employed in other body structures, including but not limited to arteries, veins, biliary ducts, urethras, fallopian tubes, bronchial tubes, the trachea, the esophagus and the prostate.

In another embodiment, the invention is also directed to vena cava filters and other medical implants formed of the alloys disclosed herein as well as to vena cava filters and other medical implants portions of which or the entirety of which are formed of relatively pure molybdenum. Desirably, the molybdenum will be at least 99% pure by weight. More desirably, the molybdenum will be 99.9% pure. Commercially available unalloyed molybdenum may be used.

Any of the inventive devices disclosed herein may be manufactured from commercially available specimens of the metal or alloy. The metal will usually have to be annealed for a brief period of time. Typically, the annealing will be carried out at a temperature which is approximately one half of the melt temperature of the metal for a period ranging between one and fifteen minutes.

Any of the inventive medical implants disclosed herein may be provided with suitable coatings to render portions of or the entirety of the stent radiopaque and/or with drugs and/or with polymer coatings. For example, the stents may be coated with gold or other noble metals or sputtered with tantalum or other metals. Other radiopaque metals which may be used include platinum, platinum-tungsten, palladium, platinum-iridium, rhodium, tantalum, or alloys or composites of these metals. Some of these metals, however, may impact the MRI compatibility of the stent.

The inventive stents or other medical devices may also be provided with various biocompatible coatings to enhance various properties of the stent or other medical device. For example, the inventive stents or other medical devices may be provided with lubricious coatings. The inventive stents or other medical devices may also be provided with drugs or drug-containing coatings which release drugs over time.

The inventive stents or other medical devices may also be provided with a sugar or more generally a carbohydrate and/or a gelatin to maintain the stent or other medical device on a balloon during delivery of the stent or other medical device to a desired bodily location. Other suitable compounds for treating the stent include biodegradable polymers and polymers which are dissolvable in bodily fluids. Portions of the interior and/or exterior of the stent or other medical devices may be coated or impregnated with the compound. Mechanical retention devices may also be used to maintain the stent or other medical device on the balloon during delivery. To that end, the use of other coatings on the inventive stents or other medical devices is also within the scope of the invention.

The coating may comprise one or more non-genetic therapeutic agents, genetic materials and cells and combinations thereof as well as other polymeric coatings.

Non-genetic therapeutic agents include anti-thrombogenic agents such as heparin, heparin derivatives, urokinase, and PPack (dextrophenylalanine proline arginine chloromethylketone) ; anti-proliferative agents such as enoxaprin, angiopeptin, or monoclonal antibodies capable of blocking smooth muscle cell proliferation, hirudin, and acetylsalicylic acid; anti-inflammatory agents such as dexamethasone, prednisolone, corticosterone, budesonide, estrogen, sulfasalazine, and mesalamine ; antineoplastic/antiproliferative/anti-miotic agents such as paclitaxel, 5-fluorouracil, cisplatin, vinblastine, vincristine, epothilones, endostatin, angiostatin and thymidine kinase inhibitors; anesthetic agents such as lidocaine, bupivacaine, and ropivacaine ; anti-coagulants such as D-Phe-Pro-Arg chloromethyl keton, an RGD peptide-containing compound, heparin, antithrombin compounds, platelet receptor antagonists, anti- thrombin anticodies, anti-platelet receptor antibodies, aspirin, prostaglandin inhibitors, platelet inhibitors and tick antiplatelet peptides; vascular cell growth promotors such as growth factor inhibitors, growth factor receptor antagonists, transcriptional activators, and translational promotors ; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; and agents which interfere with endogenous vascoactive mechanisms.

Genetic materials include anti-sense DNA and RNA, DNA coding for, anti- sense RNA, tRNA or rRNA to replace defective or deficient endogenous molecules, angiogenic factors including growth factors such as acidic and basic fibrobiast growth factors, vascular endothelial growth factor, epidermal growth factor, transforming growth factor ocand p, platelet-derived endothelial growth factor, platelet-derived growth factor, tumor necrosis factor oc, hepatocyte growth factor and insulin like growth factor, cell cycle inhibitors including CD inhibitors, thymidine kinase ("TK") and other agents useful for interfering with cell proliferation the family of bone morphogenic proteins ("BMP"s"), BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Desirable BMP"s are any of BMP-2, BMP-3, BMP-4, BMP-5, BMP-6 and BMP-7. These dimeric proteins can be provided as homodimers, heterodimers, or combinations thereof, alone or together with other molecules. Alternatively or, in addition, molecules capable of inducing an upstream or downstream effect of a BMP can be provided. Such molecules include any of the"hedgehog"proteins, or the DNA"s encoding them.

Cells can be of human origin (autologous or allogeneic) or from an animal source (xenogeneic), genetically engineered if desired to deliver proteins of interest at the transplant site. The cells may be provided in a delivery media. The delivery media may be formulated as needed to maintain cell function and viability.

Suitable polymer coating materials include polycarboxylic acids, cellulosic polymers, including cellulose acetate and cellulose nitrate, gelatin, polyvinylpyrrolidone, crosslinked polyvinylpyrrolidone, polyanhydrides including maleic anhydride polymers, polyamides, polyvinyl alcohols, copolymers of vinyl monomers such as EVA, polyvinyl ethers, polyvinyl aromatics, polyethylene oxides, glycosaminoglycans, polysaccharides, polyesters including polyethylene terephthalate, polyacrylamides, polyethers, polyether sulfone, polycarbonate, polyalkylenes including polypropylene, polyethylene and high molecular weight polyethylene, halogenated polyalkylenes including polytetrafluoroethylene, polyurethanes, polyorthoesters, proteins, polypeptides, silicones, siloxane polymers, polylactic acid, polyglycolic acid, polycaprolactone, polyhydroxybutyrate valerat and blends and copolymers thereof, coatings from polymer dispersions such as polyurethane dispersions (for example, BAYHDROL^{®}), fibrin, collagen and derivatives thereof, polysaccharides such as celluloses, starches, dextrans, alginates and derivatives, hyaluronic acid, squalene emulsions. Polyacrylic acid, available as HYDROPLUS^{®} (Boston Scientific Corporation, Natick, Mass.), and described in U. S. Pat. No. 5,091, 205, the disclosure of which is hereby incorporated herein by reference, is particularly desirable. Even more desirable is a copolymer of polylactic acid and polycaprolactone.

The inventive stents may also be used as the framework for a graft. Suitable coverings include nylon, collagen, PTFE and expanded PTFE, polyethylene terephthalate and KEVLAR, any of the materials disclosed in US 5,824, 046 and US 5,755, 770 and biograft materials. Examples of biograft materials include, but are not limited to, fibrin, collagen, elastin and hyaluronic acid. More generally, any known graft material may be used including synthetic polymers such as polyethylene, polypropylene, polyurethane, polyglycolic acid, polyesters, polyamides, their mixtures, blends and copolymers.

Suitable stent delivery devices such as those disclosed in US 6,123, 712, US 6,120, 522 and US 5,957, 930 may be used to deliver the inventive stents to the desired bodily location. The choice of delivery device will depend on whether a self-expanding or balloon expandable stent is used. The inventive stents may be delivered in conjunction with one or more stent retaining sleeves. An example of stent retaining sleeves is disclosed in US provisional application 60/238178.

The above disclosure is intended to be illustrative and not exhaustive. This description will suggest many variations and alternatives to one of ordinary skill in this art. All these alternatives and variations are intended to be included within the scope of the claims where the term "comprising" means "including, but not limited to". Those familiar with the art may recognize other equivalents to the specific embodiments described herein which equivalents are also intended to be encompassed by the claims.

Further, the particular features presented in the dependent claims can be combined with each other in other manners within the scope of the invention such that the invention should be recognized as also specifically directed to other embodiments having any other possible combination of the features of the dependent claims. For instance, for purposes of claim publication, any dependent claim which follows should be taken as alternatively written in a multiple dependent form from all prior claims which possess all antecedents referenced in such dependent claim if such multiple dependent format is an accepted format within the jurisdiction (e. g. each claim depending directly from claim 1 should be alternatively taken as depending from all previous claims). In jurisdictions where multiple dependent claim formats are restricted, the following dependent claims should each be also taken as alternatively written in each singly dependent claim format which creates a dependency from a prior antecedent-possessing claim other than the specific claim listed in such dependent claim below (e. g. claim 3 may be taken as alternatively dependent from claim 1; claim 4 may be taken as alternatively dependent on claim 2, or on claim 1; etc.).

The disclosure is intended to be illustrative and not exhaustive. This description will suggest many variations and alternatives to one of ordinary skill in this art. All these alternatives and variations are intended to be included within the scope of the attached claims. Those familiar with the art may recognize other equivalents to the specific embodiments described herein which equivalents are also intended to be encompassed by the claims attached hereto.

Although the present invention is defined in the attached claims, it should be understood that the present invention can also (alternatively) be defined in accordance with the following embodiments:
1. A medical implant for implantation in a bodily vessel, the implant having a flow passage therethrough, the implant comprising a tungsten-rhenium alloy.
2. The medical implant of embodiment 1 in the form of a stent.
3. The medical implant of embodiment 2 wherein the tungsten-rhenium alloy consists essentially of tungsten and rhenium.
4. The medical implant of embodiment 3 wherein the tungsten is present in an amount ranging from about 75 weight percent to about 99 weight percent.
5. The medical implant of embodiment 3 wherein the tungsten is present in an amount ranging from about 75 weight percent to about 99 weight percent.
6. The medical implant of embodiment 5 wherein the rhenium is present in an amount ranging from about 25 weight percent to about 1 weight percent.
7. The medical implant of embodiment 1 wherein the modulus of elasticity is at least 400 GPa.
8. The medical implant of embodiment 2 having a sidewall and a plurality of openings therein, the implant having been made from a sheet of material or from a tube.
9. A medical implant for implantation in a bodily vessel, the implant having a flow passage therethrough, the implant comprising molybdenum TZM alloy.
10. The medical implant of embodiment 9 in the form of a stent.
11. The medical implant of embodiment 9 wherein the modulus of elasticity is at least 300 GPa.
12. The medical implant of embodiment 9 having a sidewall and a plurality of openings therein, the implant having been made from a sheet of material or from a tube.
13. A stent manufactured from a sheet of metal or from a metal tube, the metal having a modulus of elasticity of 300 GPa or greater.
14. The stent of embodiment 13 wherein the modulus of elasticity is 400 GPa or greater.
15. The stent of embodiment 14 wherein the stent comprises a tungsten-rhenium alloy.
16. The stent of embodiment 13 wherein the metal consists essentially of molybdenum.
17. The stent of embodiment 13 wherein the metal consists essentially of molybdenum TZM alloy.
18. The stent of embodiment 13 comprising molybdenum TZM alloy.
19. The stent of embodiment 13 comprising molybdenum.
20. The stent of embodiment 14 comprising a tungsten-rhenium alloy.
21. The stent of embodiment 14 consisting essentially of a tungsten-rhenium alloy.
22. The stent of embodiment 21 wherein the tungsten is present in an amount ranging from about 75 weight percent to about 99 weight percent.
23. A medical implant comprising TZM alloy.
24. The medical implant of embodiment 23 in the form of a stent.

## Claims

1. A medical implant for implantation in a bodily vessel, the implant having a flow passage therethrough, the implant comprising molybdenum or a molybdenum TZM alloy.

2. The medical implant of claim 1 in the form of a stent or of a bifurcated stent.

3. The medical implant of claim 2 wherein the medical implant is a stent manufactured from a sheet of metal or from a metal tube, the metal having a modulus of elasticity of 300 GPa or greater, preferably 400 GPa or greater.

4. The medical implant of claim 3 wherein the metal consists essentially of molybdenum or essentially of molybdenum TZM alloy.

5. The medical implant of claim 2, wherein the stent has a flowpath therethrough in the direction of a longitudinal axis of the stent and a sidewall with a plurality of openings therethrough.

6. The medical implant of any one of the preceding claims wherein the molybdenum TZM alloy is a molybdenum-based alloy that contains:
between 0.25% and 1.0% titanium by weight,
between 0.04% and 2.0% zirconium by weight, and
between 0.01 % to 0.04% carbon by weight.

7. The medical implant of any one of the preceding claims, wherein the implant in its entirety is made of molybdenum or of the molybdenum TZM alloy.

8. The medical implant of any one of the preceding claims 1-6, wherein only selected portions of the implant are made of molybdenum or of the molybdenum TZM alloy with other portions of the implant made from other materials.

9. The medical implant of any one of the preceding claims comprising a coating applied to portions of or the entirety of the stent.

10. The medical implant of claim 9, wherein the implant is a stent coated with one radiopaque metal.

11. The medical implant of claim 9 or 10, wherein the implant is a stent coated with one metal selected in the group comprising: gold, tantalum, platinum, platinum- tungsten, palladium, platinum-iridium, rhodium, tantalum, alloys or composites of these metals.

12. The medical implant of claim 9 or 10 or 11, wherein the implant is coated with lubricious coatings or with drugs or with drug-containing coatings, or with a carbohydrate and/or a gelatin or with biodegradable polymers and polymers which are dissolvable in bodily fluids.

13. The medical implant of claim 9 or 10 or 11 or 12, wherein the coating may comprise one or more non-genetic therapeutic agents, or genetic materials, or cells or combinations thereof, or polymeric coatings.

14. The medical implant of any one the preceding claims 8-13, wherein the implant is a stent and wherein one or more portions of the stent may be made with one modulus of elasticity and one or more other portions of the stent may be made with a different modulus of elasticity.

15. The medical implant of the preceding claim, wherein one or both ends of the stent may be of a higher or lower modulus of elasticity than the middle portion of the stent; or wherein the stent is constructed and arranged to have an increasing modulus of elasticity along the length of the stent.
